# FASCICULE DE BREVET EUROPEEN

(11) **EP 2 456 354 B1**
(45) Date de publication et mention de la délivrance du brevet: **23.03.2016**
(21) Numéro de dépôt: 10752059.5
(22) Date de dépôt: 20.07.2010
(51) Int. Cl.: A61B 5/00

(54) **SONDE A AIGUILLE FIBREE TRANCHANTE POUR LE DIAGNOSTIC OPTIQUE EN PROFONDEUR DE TUMEURS PAR FLUORESCENCE ENDOGENE.**
SPITZE FASERNADELSONDE FÜR OPTISCHE TIEFENDIAGNOSE VON TUMOREN MITTELS ENDOGENER FLUORESZENZ
SHARP FIBROUS NEEDLE PROBE FOR THE IN-DEPTH OPTICAL DIAGNOSIS OF TUMORS BY ENDOGENOUS FLUORESCENCE

(30) Priorité: 20.07.2009 FR 0955033
(43) Date de publication de la demande: 30.05.2012
(73) Titulaire: UNIVERSITE PARIS-SUD 11, 91400 Orsay (FR); Centre National de la Recherche Scientifique, 75016 Paris (FR)
(72) Inventeur: AL CHAB, Lama, ALEP (SY); FARCY, René, Alfred, F-91370 Verrieres Le Buisson (FR); DUPUIS, Guillaume, F-75005 Paris (FR); FONTAINE-AUPART, Marie-Pierre, F-94260 Fresnes (FR)
(74) Mandataire: Pontet Allano & Associes
(86) Numéro de dépôt international: PCT/FR2010/051533
(87) Numéro de publication internationale: WO 2011/010063

(56) Documents cités:
- WO-A1-2006/044973
- WO-A2-03/020119
- US-A- 5 280 788
- US-A- 6 088 106
- US-A1- 2005 113 658
- US-A1- 2008 029 711
- US-B1- 6 174 291
- US-B1- 7 428 048
- PALASZ ZBIGNIEW ET AL: "Investigation of normal and malignant laryngeal tissue by autofluorescence imaging technique." AURIS, NASUS, LARYNX DEC 2003 LNKD- PUBMED:14656564, vol. 30, no. 4, décembre 2003 (2003-12), pages 385-389, XP002604888 ISSN: 0385-8146 DOI: 10.1016/j.anl.2003.07.012 & DATABASE MEDLINE [Online] US NATIONAL LIBRARY OF MEDICINE (NLM), BETHESDA, MD, US; décembre 2003 (2003-12), PALASZ ZBIGNIEW ET AL: "Investigation of normal and malignant laryngeal tissue by autofluorescence imaging technique." Database accession no. NLM14656564
- LEHTINEN R ET AL: "Penetration of disposable needles", INTERNATIONAL JOURNAL OF ORAL SURGERY, MUNKSGAARD, COPENHAGEN, DK, vol. 8, no. 2, 1 April 1979 (1979-04-01), pages 145-148, XP025578133, ISSN: 0300-9785, DOI: 10.1016/S0300-9785(79)80010-1 [retrieved on 1979-04-01]

## Description

La présente invention concerne un dispositif d'observation ou de diagnostic in vivo d'un tissu biologique compact ou d'un organe vivant, permettant d'aller mesurer la fluorescence endogène du tissu à l'aide d'une sonde optique sous cutanée. Ce dispositif comprend une sonde incluant une aiguille à pointe tranchante entourant une fibre optique connectée à un dispositif d'injection et de récupération de lumière pour mesurer la rétrodiffusion de la lumière injectée et la fluorescence endogène générée par les tissus sous l'effet de cette lumière injectée.

### Domaine et état de la technique

Pour le diagnostic d'une tumeur, par exemple dans le cas du sein, une première étape est souvent la découverte d'une grosseur suspecte par palpation ou d'une tâche anormale sur une mammographie de dépistage systématique. Ces méthodes initiales sont peu couteuses et présentent peu ou pas de risques pour la santé, et peuvent donc être pratiquées couramment et facilement dans un environnement de médecine de ville. Dans le cas d'une telle alerte, on a recours à des examens complémentaires pour obtenir plus de certitude sur la nature de l'anomalie découverte, en particulier pour évaluer les probabilités qu'il s'agisse d'une tumeur maligne ou bénigne, voire même d'un simple kyste. De tels examens complémentaires permettent de prendre une décision quant à la nécessité et la nature d'un traitement, qu'il s'agisse par exemple d'une opération chirurgicale d'ablation, d'un traitement par chimiothérapie ou par radiothérapie.

Or les examens complémentaires actuellement disponibles, bien que moins lourds que les traitements, sont eux-mêmes sources de contraintes, de coûts et de désagréments. Il s'agit en général d'examens d'imagerie générale souvent à base de rayonnements irradiants voire avec injection de produits traceurs eux-mêmes irradiants, par exemple une scintigraphie. Il peut s'agir aussi d'examens invasifs tels qu'une biopsie par prélèvement des tissus concernés pour réaliser en laboratoire une analyse histologique de la partie suspecte.

Ces types d'examens sont souvent coûteux et nécessitent un déplacement en environnement spécialisé pour ce qui est de l'imagerie générale. Les prélèvements nécessitent en outre le plus souvent un acte invasif voire chirurgical, qui est aussi source de traumatisme et nécessite des conditions opératoires spécifiques et contraignantes. Les contraintes liées à ces examens complémentaires font qu'ils ne sont pratiqués qu'à partir d'un certain seuil de suspicion.

Il est donc intéressant de pouvoir effectuer des examens plus simples et moins coûteux rapidement après la découverte d'une alerte, permettant une première sélection parmi les différents cas d'alerte, et ce dans des conditions moins contraignantes et plus systématiques.

Dans le cas des organes internes, des examens optiques sont pratiqués par endoscopie, par les voies naturelles ou par cathéter introduit dans des vaisseaux ou des artères, qui permettent d'analyser par spectroscopie la nature des cellules externes de l'organe visé. Ces systèmes, dits parfois de biopsie optique, utilisent des sondes comprenant un faisceau de fibres optiques pour transporter la lumière depuis l'intérieur du corps jusqu'à l'opérateur ou à un appareil d'enregistrement, et obtenir ainsi une image en deux dimensions de la surface de l'organe observé, comme par exemple dans le document WO/2006/000704.

Ces systèmes multifibres utilisent parfois des techniques de mesure de la fluorescence des cellules sous une excitation lumineuse, comme par exemple dans le document US 5,562,100 ou bien US 5,303,026. D'autres appareils utilisent des sondes d'imagerie qui viennent au contact de la surface externe des organes, ou qui restent légèrement à distance de cette surface comme dans le document WO/2008/020130 qui décrit une fibre sortant de la sonde pour pénétrer partiellement dans une alvéole pulmonaire pour en visualiser les parois intérieures.

Cependant, depuis l'extérieur du corps comme en endoscopie, il existe peu de solutions simples permettant une analyse des tissus situés à l'intérieur, c'est à dire derrière la muqueuse d'un organe compact comme un ganglion, ou à l'intérieur de la paroi c'est à dire entres les muqueuses d'un organe creux comme le coeur ou les poumons, ou simplement derrière l'épiderme comme un muscle ou un sein.

Certains instruments optiques ont été développés, qui sont prévus pour être enfoncés dans les tissus et effectuer différentes analyses. On peut citer pour cela le document EP 0 483 618 qui décrit une aiguille contenant un plastique transparent permettant de visualiser la couleur du sang du côté proximal lorsque l'on pénètre dans une veine.

Certains documents tels que US4566438, US4269192, WO 9214399A1 décrivent une aiguille tranchante intégrant plusieurs fibres optiques pour analyser un tissu. Le brevet EP0635238 intègre une fibre monomode dans une sonde pointue partiellement transparente pour effectuer de la Tomographie Optique Cohérente (OCT), ou le brevet US20050027199 insérant des fibres dans des outils tranchant toujours pour effectuer des mesures d'OCT. Le brevet WO 2008068685 décrit un dispositif constitué d'une sonde multifibres intégrée dans une aiguille de biopsie pour la spectroscopie, le brevet EP 0513986 propose un dispositif à fibre intégré dans une sonde tranchante pour mesurer la fluorescence exogène induite par des photosensibilisateurs (photofrin) dans une tumeur. Le brevet WO 03/020119 A2 décrit un dispositif sous cutané à aiguille multi capteurs et multifibres positionné en profondeur destiné à faire des mesures de diffusion, de pO₂, de spectrométrie d'absorption, d'impédance électrique, de température.

Ces dispositifs utilisent tous des moyens d'analyse complexes et coûteux, ce qui limite les possibilités de diffusion du système.

Aucun des dispositifs sous cutanés à aiguille décrits ci-dessus ne prévoit ni ne permet de détecter le très faible signal de fluorescence endogène de la tumeur de façon satisfaisante, en particulier dans un dispositif de diamètre très faible tel celui des aiguilles les plus fines (gauge 23G et 25G). Leur usage est donc aussi invasif qu'une biopsie, et présente donc peu d'intérêt en utilisation préalable à une biopsie, par exemple en tant que dépistage ou comme confirmation lors en cas de doute à l'interprétation d'une radiologie de dépistage.

De plus, ces appareils présentent une structure de sonde qui est souvent trop complexe pour être facilement stérilisés en autoclave. Or la complexité de ces sondes fait qu'elles sont trop coûteuses à produire pour être considérées comme jetables, en particulier dans l'optique d'un test de dépistage ou préopératoire utilisé sur une population très nombreuse.

Un but de l'invention est fournir des moyens et des méthodes permettant d'effectuer des examens plus simples, plus rapides et moins coûteux, par exemple en dépistage complémentaire de l'échographie après radiologie, en limitant les contraintes d'environnement, de déplacement, de temps, mais aussi de compétences de l'opérateur. Il est ainsi recherché de fournir des moyens d'examens complémentaires accessibles en cabinet médical de spécialiste ou dans un centre de radiologie non hospitalier.

Un autre but de l'invention est de fournir un moyen d'explorer la fluorescence endogène, signal très faible à l'intérieur des organes pleins par une aiguille de diamètre minimal.

Un autre objet de la présente invention est la réalisation d'une sonde optique fibrée pour le diagnostic optique de fluorescence endogène en profondeur des tumeurs.

### Exposé de l'invention

L'invention propose un dispositif d'observation ou de diagnostic in vivo par fluorescence endogène d'un tissu biologique compact ou d'un organe vivant, comprenant une sonde munie d'une extrémité proximale du côté de l'opérateur et une extrémité distale du côté du tissu à observer. Ce dispositif est défini dans la revendication 1.

Selon l'invention, cette sonde comprend :
- une aiguille creuse se terminant à l'extrémité distale par une pointe coupante apte à pénétrer la surface du tissu à observer et à s'enfoncer à l'intérieur dudit tissu ;
- entourant de façon solidaire une fibre optique unique dont l'extrémité proximale est connectée ou est agencée pour être connectée à des moyens d'injection et de récupération de lumière, et dont l'extrémité distale présente un profil transversal dans la continuité de la pointe de l'aiguille.

Le dispositif comprend en outre :
- des moyens d'injection et de récupération de lumière agencés pour injecter un signal lumineux, dit aller, dans l'extrémité proximale de la fibre optique, et recevoir de l'extrémité distale de la fibre optique unique un signal lumineux dit retour de rétrodiffusion et de fluorescence endogène générée par l'excitation due à ladite lumière injectée ;
- un dispositif d'analyse pour mesurer ledit signal retour ; et
- des moyens de télémétrie optique fixés sur la partie extérieure de ladite sonde et agencés pour mesurer en temps réel et transmettre la profondeur d'enfoncement de ladite sonde à l'intérieur du tissu à partir d'une mesure de la distance jusqu'à la surface extérieure dudit tissu.

De préférence, la fibre optique est du type multimode, ce qui apporte de meilleures performances, par exemple pour meilleure sensibilité et facilité de réglage.

Selon les modes de réalisation, le dispositif d'analyse peut être, par exemple de spectrophotométrie tel que connu dans l'état de la technique.

Il peut aussi être réalisé par un dispositif mesurant uniquement la seule intensité du signal de fluorescence endogène, par exemple pour la comparer à l'intensité de la partie rétrodiffusée de la lumière d'excitation.

En piquant l'aiguille dans l'organe visé, par exemple dans le sein à travers la peau, l'invention permet d'évaluer la nature des cellules situées à la pointe de l'aiguille.

Cette aiguille présente des dimensions et des caractéristiques similaires à celles des aiguilles de type standard, de préférence les plus fines, utilisées pour des injections ou des ponctions courantes comme des vaccins ou des prises de sang.

Il est ainsi possible de réaliser dans un contexte médical simple et de proximité une première analyse des cellules suspectes même à l'intérieur d'un organe ou derrière la peau.

Dans un mode de réalisation, les moyens d'injection et de récupération de lumière sont agencés pour être connectés à un spectrophotomètre agencé pour observer la partie du tissu située au contact de l'extrémité distale de la fibre optique, en mesurant à l'extrémité proximale de cette fibre la rétrodiffusion de la lumière injectée et la fluorescence endogène générée par l'excitation due à ladite lumière injectée.

Selon une particularité, le dispositif d'analyse est agencé pour être connecté à des moyens de calcul numériques agencés pour réaliser une analyse de l'intensité, ou de la durée, ou de la longueur d'onde de la lumière rétrodiffusée et de la fluorescence générée, ou une combinaison de ces trois grandeurs.

Dans un mode de réalisation, les moyens d'injection de lumière injectent une lumière d'une longueur d'onde comprise entre le proche infra rouge, par exemple un micromètre voire 1,6 ou 2 µm et le proche ultra violet, par exemple 370 voire 300 ou 250 nanomètres.

Par exemple, les moyens d'injection de lumière comprennent au moins une source de lumière laser, telle qu'une diode laser à 405 nm, pour générer la lumière injectée.

Selon une autre particularité, le dispositif d'analyse est agencé pour être connecté à des moyens de calcul numériques agencés pour réaliser une analyse de l'intensité, et possiblement de la longueur d'onde de la fluorescence générée,

Dans un mode de réalisation préféré, les moyens d'injection de lumière injectent une lumière d'une longueur d'onde comprise entre 370 et 420 nanomètres. La puissance de la diode laser est choisie telle que la puissance en sortie de fibre se situe à la limite autorisée en fonction des normes de sécurité laser sur la peau.

Par exemple, les moyens d'injection de lumière comprennent une unique source de lumière laser, telle qu'une diode laser à 405 nm, pour générer la lumière injectée. Cette lumière est focalisée par un objectif vers l'entrée de la fibre après être passée au travers d'un élément séparateur, par exemple un cube ou une lame.

Le signal de retour provenant de la même fibre unique est collecté par le même objectif ayant servi à l'injection, puis redirigé vers l'élément séparateur qui le renvoie vers le dispositif d'analyse.

Dans une première variante, le dispositif d'analyse comprend un filtre interférentiel coupant la longueur d'onde d'excitation et laissant passer les longueurs d'ondes supérieures et un objectif focalisant la lumière sur l'entrée d'un spectrophotomètre refroidi.

Dans une seconde variante, le dispositif d'analyse comprend une lame dichroïque, à longueur d'onde de séparation légèrement supérieure à celle de la lumière injectée, séparant le signal en deux voies. La lumière de la voie correspondant à la longueur de la lumière injectée est focalisée sur une photodiode, et la lumière de la voie correspondant aux longueurs d'onde de la fluorescence est focalisée sur une photodiode à avalanche. Les deux signaux sont ensuite traités électroniquement.

### Configuration de l'aiguille

L'utilisation comme signal de la fluorescence endogène, ou auto fluorescence, permet d'éviter l'emploi de produits spécifiques pour renforcer cette fluorescence, qui sont souvent des produits délicats à manier car sources d'effets secondaires indésirables pour le patient.

Or cette fluorescence endogène produit par nature un signal très faible, qui est donc difficile à détecter et à mesurer.

Nombre d'aiguilles médicales existantes pour différents types d'injections présentent un biseau de 15° ou de 30°. L'angle de 90° correspondant ainsi à une extrémité plate et perpendiculaire à l'axe de l'aiguille.

De manière préférée, l'extrémité distale de l'aiguille et de la fibre optique présente un profil transversal en un biseau simple faisant avec l'axe de l'aiguille un angle compris entre 10° et 25° voire 20°, et notamment de 15°. Il est à noter que la notion de biseau « simple » correspond à un biseau avec une seule surface principale, mais n'exclut pas des légers affutages latéraux de la partie métallique autour de la pointe.

En plus de l'affûtage, l'utilisation d'un angle plutôt aigu permet en effet de diminuer la résistance à la pénétration et de limiter la douleur causée. Il permet aussi de récupérer un signal suffisant dans une aiguille de diamètre minimal car il est nécessaire de présenter la plus grande surface possible d'interaction.

Selon une autre particularité, la sonde comporte en outre une résine réunissant en une même surface continue et étanche l'extrémité distale de la fibre optique avec la pointe de l'aiguille.

On évite ainsi les risques d'effritement de la pointe de la fibre qui pourrait alors laisser des particules à l'intérieur du corps du patient au moment du retrait de l'aiguille.

De manière préférée on utilisera les aiguilles les plus fines pour réduire la douleur, soit de 23G ou 25G.

La fibre à l'intérieur de l'aiguille sera de préférence une fibre silice, de préférence une silice optimisée pour la transmission dans le bleu et le vert, par exemple dans la bande 400-600 nm, et de dimension de coeur de l'ordre de 200 µm pour une aiguille de 25G.

L'ensemble de l'aiguille et de la fibre sont ainsi suffisamment simples et économiques à fabriquer pour pouvoir être conçu comme dispositif à usage unique, ce qui permet d'éviter que le tranchant s'émousse lors d'usages multiples.

### Mesures multiples

Un autre but de l'invention est de fournir un dispositif permettant une analyse précise et sur une certaine zone au sein des tissus concernés, tout en limitant le nombre de perforations, et donc les traumatismes et la douleur.

Pour cela, l'invention propose de réaliser plusieurs mesures au cours d'une même opération de perforation, point par point sur toute la profondeur explorée par l'aiguille.

Un objet de la présente invention est alors aussi de permettre de fournir un signal de diagnostic de façon précise à plusieurs profondeurs différentes sur la même trajectoire.

Pour cela, le dispositif selon l'invention comprend en outre des moyens de télémétrie optique fixés sur la partie extérieure de la sonde et agencés pour mesurer et transmettre la profondeur d'enfoncement de ladite sonde à l'intérieur du tissu à partir d'une mesure de la distance jusqu'à la surface extérieure dudit tissu.

En outre, les moyens de calcul numériques peuvent être agencés pour déclencher et enregistrer une pluralité de mesures à une pluralité de profondeurs différentes au cours d'un déplacement de la sonde selon l'axe longitudinal de l'aiguille, et pour corréler l'analyse de cette pluralité de mesures avec ladite pluralité de profondeurs.

La sonde peut ainsi être utilisée pour réaliser plus facilement une pluralité de mesures à une pluralité de profondeurs différentes lors d'une même introduction de la sonde à travers la peau ou la surface de l'organe visé, de préférence au cours de l'extraction. En réalisant automatiquement une mesure pour chaque pas de profondeur au cours d'un déplacement, ou une moyenne de plusieurs successives pour chaque profondeur, on obtient une cartographie linéaire d'analyse des tissus sur toute la profondeur d'enfoncement. La corrélation automatique des moyens de télémétrie avec l'enregistrement des mesures permet de retirer l'aiguille sans contrainte particulière quant la régularité ou la vitesse du mouvement de retrait.

Selon une particularité, les moyens de télémétrie optique comprennent au moins une paire de fibres optiques juxtaposées, parmi lesquelles une première fibre de télémétrie envoie parallèlement à l'axe de l'aiguille un faisceau de lumière vers la surface extérieure du tissu à observer, et une seconde fibre de télémétrie récupère la lumière rétrodiffusée par ladite surface, et est connectée à un phototransistor ou une photodiode qui mesure la fraction rétrodiffusée par la surface au sein du flux de lumière émise par la première fibre de télémétrie, fournissant ainsi une mesure de la distance jusqu'à ladite surface extérieure.

Plus particulièrement, les moyens de télémétrie peuvent comprendre un télémètre optique à triangulation, par exemple laser.

Les différentes caractéristiques exposées plus haut peuvent être combinées entre elles de différentes façons, et leurs différentes combinaisons sont ici explicitement évoquées et spécifiées.

### Applications

Dans des applications typiques, telles que l'analyse d'anomalies mammaires, l'invention peut être mise en oeuvre dans des modes de réalisation où la sonde est conformée pour une utilisation par l'extérieur du corps pour l'observation ou le diagnostic in vivo des tissus ou organes par enfoncement à travers l'épiderme.

La sonde peut alors présenter des dimensions pour le diamètre extérieur de l'aiguille 3 et respectivement pour le diamètre extérieur de la fibre 2 inférieures à 2,10 mm et respectivement 1,50 mm, soient compatibles avec une aiguille standard de type gauge 14. Plus particulièrement les dimensions de l'aiguille et de la fibre pourront être inférieures à 1,30 mm et respectivement 0,80 mm, c'est à dire compatibles avec une aiguille standard de type gauge 18 ou plus.

Des dimensions correspondant aux aiguilles de 23 ou 25G constituent une solution intéressante.

Le dispositif permet d'effectuer un diagnostic optique en profondeur des tumeurs dans le domaine de la cancérologie, avec l'avantage de limiter l'aspect invasif de la procédure, d'une bonne biocompatibilité, en limitant le temps de mise en oeuvre et les compétences de la part de l'opérateur, ainsi que pour un coût limité. Il est possible par exemple d'envisager une sonde utilisant des aiguilles jetables, ou une sonde jetable en elle-même.

L'invention permet ainsi un procédé d'examen et d'analyse d'une zone suspectée d'être une tumeur, par exemple une tumeur mammaire.

La tumeur sera explorée par exemple avec une aiguille de gauge 18 ou 23 ou plus, contenant une fibre de diamètre 200µm de coeur, voire 730 µm ou moins. L'aiguille sera d'une longueur de l'ordre de 10 cm au moins pour satisfaire aux besoins de l'exploration mammaire. La tumeur sera localisée à l'échographie de façon similaire aux procédures de biopsies par aiguille fine, et on utilisera l'aiguille et le signal optique pour explorer la zone tumorale et une zone saine pour comparaison.

Une procédure pourra être d'insérer l'aiguille au travers de la tumeur de façon rapide pour limiter la douleur, et de contrôler la position de l'aiguille à l'échographie. Ensuite, on retire lentement l'aiguille. Les mesures sont prises en permanence et organisées grâce au dispositif télémétrique, par exemple à une vitesse d'environ 5 secondes par centimètre pour une durée d'acquisition de 100 ms à 500 ms. On obtiendra ainsi une résolution spatiale comprise entre 0,2 mm et 1 mm. Les données seront affichées et enregistrées en temps réel, en même temps que les images vidéo de l'échographie.

Dans d'autres applications, il peut aussi être envisagé de mettre en oeuvre l'invention dans des modes de réalisation où la sonde est conformée pour une utilisation endoscopique pour l'observation ou le diagnostic in vivo de l'intérieur de tissus ou d'organes compacts, par enfoncement à travers l'enveloppe de ces tissus ou organes depuis une voie accessible par endoscopie. Il peut s'agir par exemple d'un mode de réalisation où la sonde présente des dimensions lui permettant d'être introduite dans le canal opérateur d'un endoscope avec ses moyens de télémétrie situés à la sortie du canal opérateur. Pour cela, le mode télémétrique de mesure de la distance décrit à deux fibres est particulièrement bien adapté.

Des modes de réalisation variés de l'invention sont prévus, intégrant selon l'ensemble de leurs combinaisons possibles les différentes caractéristiques optionnelles exposées ici.

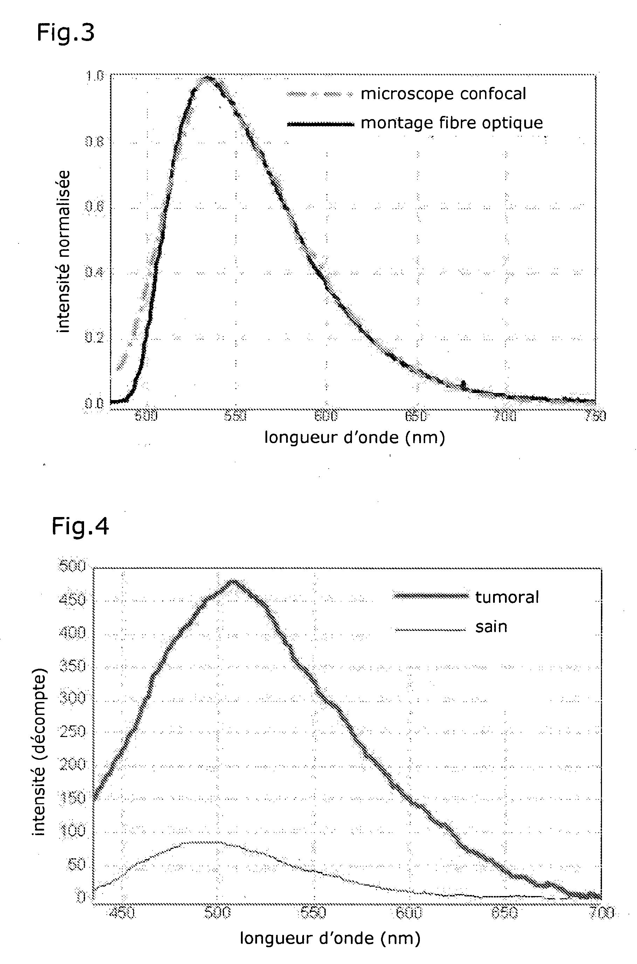

D'autres particularités et avantages de l'invention ressortiront de la description détaillée d'un mode de mise en oeuvre nullement limitatif, et des dessins annexés sur lesquels :
- la FIGURE 1 est une vue schématique en coupe illustrant un mode de réalisation de l'invention en position d'analyse dans une zone tumorale supposée située sous la peau ;
- la FIGURE 2A est un schéma illustrant l'architecture des moyens d'injection et récupération de lumière, dans un mode de réalisation de l'invention ;
- la FIGURE 2B est un schéma illustrant une variante de l'architecture du dispositif d'analyse dans un mode de réalisation de la FIGURE 2a ;
- la FIGURE 3 est un graphique expérimental d'intensité normalisée en fonction du spectre, illustrant la validité des résultats obtenus avec l'architecture de fluoroscopie endogène par fibre optique de l'invention par rapport à ceux obtenus avec un microscope confocal ;
- a FIGURE 4 est un graphique expérimental d'intensité en fonction du spectre, illustrant la validité de la détection par différence d'intensité réalisée par l'architecture de fluoroscopie endogène par fibre optique de l'invention par comparaison entre les résultats obtenus pour un tissu tumoral et un tissu sain ;

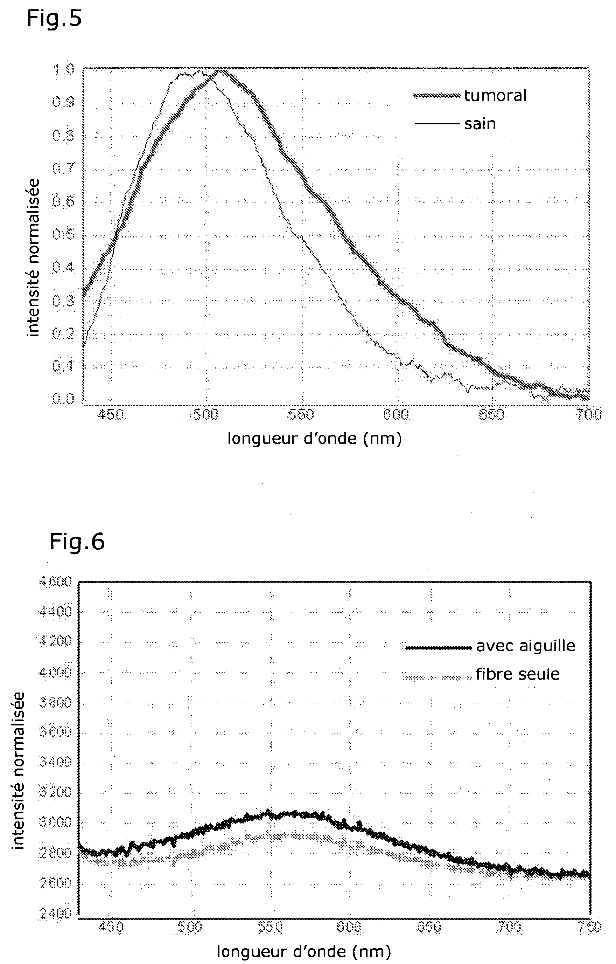

- la FIGURE 5 est un graphique expérimental d'intensité normalisée en fonction du spectre, illustrant la validité de la détection par décalage spectral réalisée par l'architecture de fluoroscopie endogène par fibre optique de l'invention par comparaison entre les résultats obtenus pour un tissu tumoral et un tissu sain ;
- la FIGURE 6 est un graphique expérimental d'intensité en fonction du spectre, illustrant les bruits de fonds obtenus dans un tissu sain avec l'architecture à aiguille et fibre aiguisée comparés avec ceux obtenus avec l'architecture avec fibre seule à extrémité droite ;

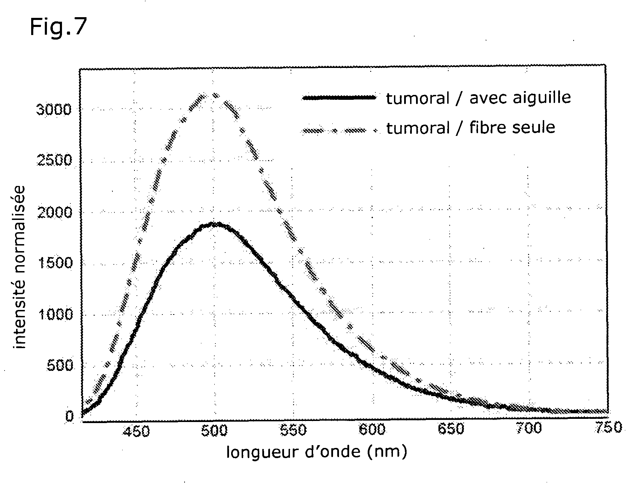

- la FIGURE 7 est un graphique expérimental d'intensité en fonction du spectre, illustrant les résultats obtenus dans un tissu tumoral avec l'architecture à aiguille et fibre aiguisée comparés avec ceux obtenus avec l'architecture avec fibre seule à extrémité droite.

### Description des figures

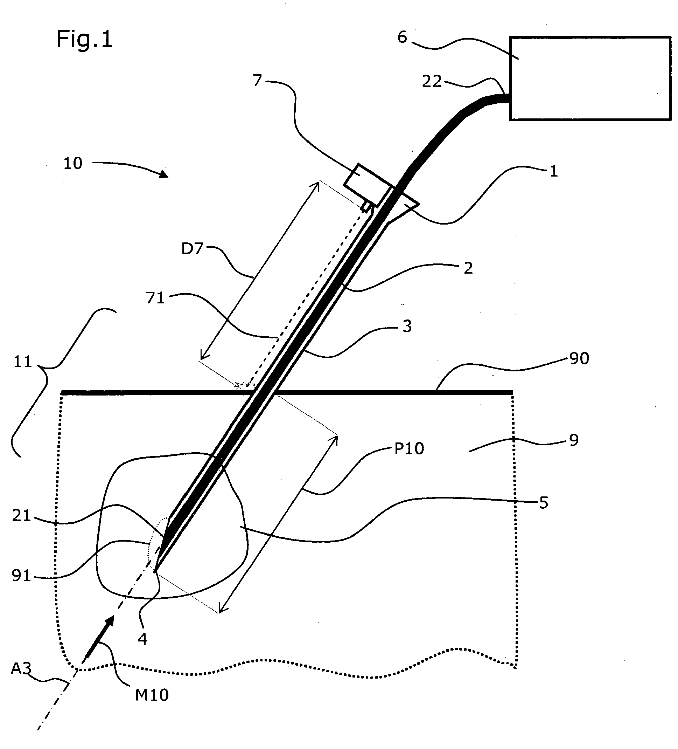

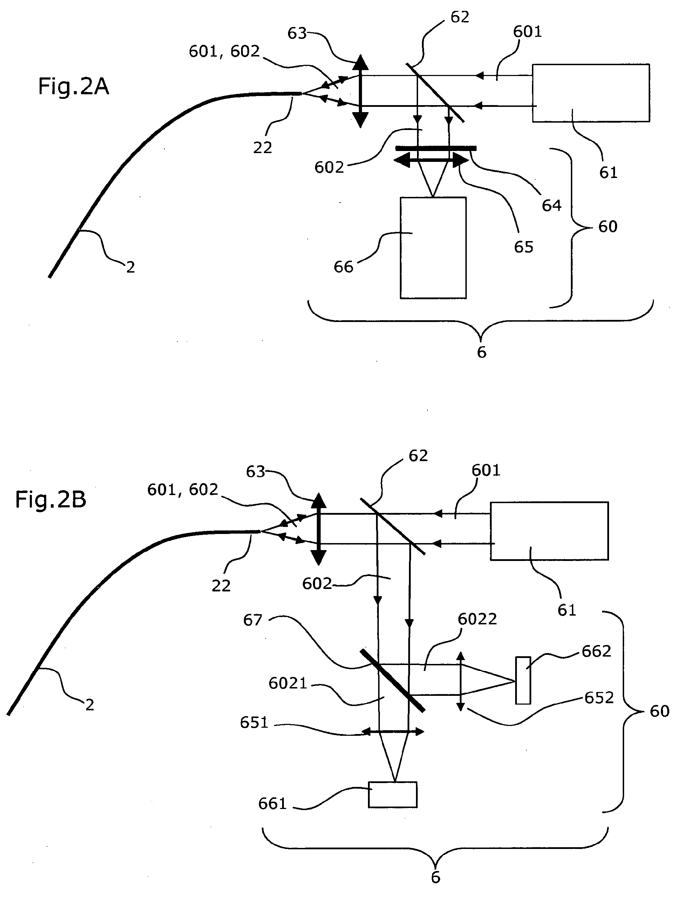

Dans le un mode de réalisation illustré en FIGURE 1 et FIGURE 2A et 2B, la sonde 10 du dispositif selon l'invention comprend à son extrémité distale 11 une fibre optique 2 incluse dans une aiguille 3 creuse se terminant par une pointe coupante 4. La fibre est collée dans le creux de l'aiguille et polie à son extrémité distale 21 de façon à épouser la forme tranchante de la pointe 4 de l'aiguille. L'angle de polissage de l'extrémité distale 21 de la fibre optique 2 correspond à celui du tranchant 4 de l'aiguille.

Un dispositif 6 d'injection et de récupération de lumière placé à l'entrée proximale 22 de la fibre injecte un signal lumineux aller 601, le tissu 91 situé à l'extrémité distale de l'aiguille et de la fibre renvoie un signal retour lumineux 602 de rétrodiffusion et de fluorescence (endogène) dans la fibre 2. Ce signal retour 602 est transmis par la fibre 2 au dispositif d'injection récupération de lumière 6 qui l'analyse, en particulier en intensité et longueur d'onde. Un télémètre optique 7 placé sur l'extérieur de l'embout proximal 1 de l'aiguille mesure la distance D7 à la peau et permet de déduire la profondeur P10 de la zone explorée 91.

Dans le mode de réalisation illustré en FIGURE 2A, le dispositif d'injection récupération de lumière 6 comprend une source de lumière 61 focalisée sur l'entrée 22 de la fibre 2 au travers d'une lame séparatrice 62, qui récupère le signal retour 602 et le renvoie vers un spectrophotomètre 66 analysant le signal retour en intensité et longueur d'onde, voire en durée.

La lumière 601 injectée dans la fibre 2 se situe dans le domaine de longueur d'onde compris entre le proche infrarouge et le proche ultraviolet et peut comprendre une ou plusieurs sources lasers, continues ou impulsionnelles.

En particulier pour l'observation par la fluorescence endogène, la lumière injectée se situe de préférence entre 370 et 420 nanomètres.

Dans une variante illustrée en FIGURE 2B, le dispositif d'analyse 60 comprend des moyens de séparation selon la longueur d'onde (ici une lame dichroïque) 67, à longueur d'onde de séparation de 5 à 30 nm supérieure à celle de la lumière injectée (soit par exemple une longueur d'onde de séparation de 420 nm), disposée pour séparer tout ou partie du signal retour 602 en deux voies :
- une première voie 6021 est formée qui comprend de la lumière correspondant aux longueurs d'onde de la fluorescence endogène des tissus observés 91 (ici la lumière qui a traversé la lame dichroïque 67), puis est focalisée par un objectif (ici une lentille 651) sur un premier photodétecteur, ici une photodiode à avalanche 661 ; et
- une deuxième voie (6022) dans laquelle la lumière correspond à la longueur de la lumière injectée 601 (ici la lumière réfléchie par la lame dichroïque), et est focalisée par un objectif (652) sur un deuxième photodétecteur, par exemple une photodiode 662.

Les deux signaux sont alors traités électroniquement et comparés par des moyens de calcul et/ou de représentation.

En filtrant ainsi le signal retour 602 selon sa longueur d'onde, il est possible de mesurer la fluorescence endogène en se contentant de mesurer sa seule intensité, ici dans la diode à avalanche 661, qui peut ensuite être comparée à l'intensité d'excitation mesurée par la photodiode 662 de la deuxième voie. On obtient en particulier un dispositif d'analyse 60 simple, compact et robuste, réalisable avec des composants peu nombreux et peu coûteux ; et bien adapté aux fluctuations rapides de signaux lors de la pénétration de l'aiguille dans la peau.

Afin de repérer la profondeur de l'aiguille le télémètre optique 7 situé à la base 1 de l'aiguille 3 comprend une paire de fibres optiques de télémétrie juxtaposées (non représentées). Une première fibre de télémétrie envoie un faisceau de 71 lumière dans l'axe A3 de l'aiguille 3. Une seconde fibre de télémétrie récupère la lumière rétrodiffusée par la peau ou la surface 90 de l'organe 9. A l'extrémité proximale de la seconde fibre de télémétrie, un détecteur comme par exemple un phototransistor mesure la fraction du flux 71 qui est rétrodiffusé par la peau 90, ce qui permet d'évaluer la distance D7 entre la peau 90 et le télémètre 7, et/ou la variation de cette distance D7. La variation de cette distance D7 permet au dispositif de mesurer et d'enregistrer la profondeur P10 d'enfoncement de la sonde à l'intérieur du tissu 9 exploré.

Selon une variante du télémètre, non représentée ici, le télémètre optique situé à la base de l'aiguille comprend un télémètre optique à triangulation, pouvant être réalisé selon différentes technologies comme par exemple à LED classique ou à laser.

Selon un mode de réalisation, le dispositif sonde 1 comprend d'une unique fibre optique 2 de diamètre extérieur 736 µm (environ), de diamètre de coeur 300 µm et d'ouverture numérique 0,22. Cette fibre est insérée dans le creux d'une aiguille 3 de type 18G (« gauge 18 »), avec une pointe coupante 4 ayant un tranchant incliné à 15°.

De préférence, selon un autre mode de réalisation, la fibre optique 2 est de diamètre extérieur 250µm, de diamètre de coeur 200 µm et d'ouverture numérique 0,22. Elle est insérée dans le creux d'une aiguille 3 de type « gauge 25 », avec une pointe coupante 4 ayant un tranchant incliné à 15°.

La fibre 2 est insérée et collée dans le creux de l'aiguille 3 avec de la colle époxy. Elle est ensuite polie de façon à épouser la forme tranchante de l'extrémité de l'aiguille. Une fine couche de colle époxy par exemple transparente est conservée en surface, possiblement sur le seul pourtour de la fibre, qui comble les espaces et préviendra de l'effritement des angles du bout de la fibre.

Le tissu ou l'organe 9 à explorer est piqué par l'aiguille 3, jusqu'à traverser la tumeur 5 à analyser.

Le dispositif d'injection récupération de lumière 6 placé à l'entrée de la fibre 2 est constitué d'une source de lumière 61 envoyant un faisceau collimaté 601 traversant la lame séparatrice 62 puis convergeant sur la face d'entrée de l'extrémité proximale 22 de la fibre 2, après traversée du premier objectif 63. La lumière retour 602 renvoyée par la tumeur repasse par le premier objectif 63 puis est déviée par la séparatrice 62 vers un filtre 64 atténuant la longueur d'onde émise par la source 61, puis est injectée grâce à un deuxième objectif 65 vers le spectrophotomètre 66.

A titre d'exemple non limitatif la source de lumière 61 peut comprendre ou être constituée d'une diode laser collimatée à 405 nm de puissance 20mW, la lame séparatrice 62 d'un cube séparateur, les objectifs 63 et 65 d'objectifs de microscope X10, le filtre 64 d'un filtre passe-haut de longueur d'onde de coupure 420nm, le spectrophotomètre 66 d'un spectrophotomètre fibré à barrette CCD refroidi par effet Peltier.

La partie 91 du tissu 5 ou 9 située à l'extrémité distale 21 et 4 de la fibre 2 et de l'aiguille 3 rétrodiffuse la lumière incidente 601 dans la fibre. Elle est en outre excitée par cette même lumière incidente 601, ce qui génère en plus un signal de fluorescence dont une partie du flux est récupérée par l'extrémité distale 21 de la fibre 2. La lumière 602 renvoyée vers le dispositif d'injection récupération 6 est analysée, en particulier en intensité, durée et longueur d'onde par le spectrophotomètre 66. Le résultat de cette analyse, ou des calculs réalisés à partir de tout ou partie de ces grandeurs, permet un diagnostic du matériau exploré en profondeur, sans prélèvement.

Ce diagnostic est basé sur les spécificités en intensité, spectre et durée du signal tumoral formant une signature dite signature tumorale, dont les caractéristiques ont été étudiées et évaluées par les inventeurs, comme décrit ci-après.

Un télémètre optique 7 est placé sur l'embout extérieur, c'est à dire la partie proximale de l'aiguille 3, et mesure la distance D7 de l'embout de l'aiguille à la peau 90. La longueur D3 de l'aiguille 3 étant connue, cela permet à des moyens de calculs (non représentés) de connaître la profondeur P10 de la zone explorée, et de fournir et enregistrer les signaux repérés chacun en fonction de sa profondeur.

L'aiguille est enfoncée rapidement pour réduire la douleur, puis retirée doucement, plus elle sera retirée lentement plus la résolution spatiale de la mesure sera bonne. En chaque point on connaît la profondeur de la zone explorée grâce au télémètre. A titre d'exemples non limitatifs le signal de diagnostic peut être constitué de l'intensité globale de fluorescence générée par une excitation à 405 nm, de la forme du spectre, et/ou de l'intensité rétro diffusée à 405nm.

### Résultats de signature spectrale

Les inventeurs ont testé un prototype correspondant au mode de réalisation décrit ici, pour valider les résultats obtenus avec ce type d'architecture et à 405 nm.

Le graphique de la FIGURE 3 représente l'intensité normalisée avec l'architecture de fluoroscopie endogène par fibre optique de l'invention, sur un domaine spectral allant de 480 nm à 750 nm, comparée avec les résultats d'un microscope confocal, qui constitue fréquemment une référence en la matière. La conformité de ces deux courbes montre que l'architecture du dispositif selon l'invention donne des résultats très proches du microscope confocal, signe favorable quant à la validité des analyses qui pourront en être tirées.

Le graphique de la FIGURE 4 représente l'intensité de fluorescence obtenue par le dispositif selon l'invention sur un domaine spectral de 430 n m à 700 nm, en moyenne pour dix mesures sur un échantillon tumoral avéré (courbe épaisse en haut) par comparaison avec un échantillon sain (courbe fine en bas). L'intensité autour du pic de 510 nm est environ cinq fois plus élevée pour l'échantillon tumoral, et constitue ainsi un critère de différentiation robuste utilisable pour établir une analyse voire un diagnostic grâce au dispositif selon l'invention.

Le graphique de la FIGURE 5 représente les intensités de la FIGURE 4 après normalisation en intensité. Cette normalisation permet de constater entre outre un décalage spectral vers le rouge allant de 10 nm à 25 nm. Ce décalage spectral constitue un autre critère de différentiation utilisable pour établir une analyse voire un diagnostic grâce au dispositif selon l'invention.

Ces deux critères apparaissent ainsi particulièrement intéressants, ensemble ou séparément, en combinaison avec l'architecture ici décrite.

Le dispositif selon l'invention peut ainsi comprendre des moyens de calcul numériques programmés ou agencé pour calculer l'un ou l'autre de ces critères ou une combinaison de ces critères. L'appareil peut alors en tirer une analyse, par exemple chiffrée ou en une représentation graphique linéaire, voire les comparer à un tableau diagnostic pour émettre un diagnostic ou une assistance au diagnostic.

### Performances de la sonde biseautée

A partir de la validation de la signature spectrale, les inventeurs ont étudié plus précisément les résultats obtenus avec une sonde aiguisée intégrée dans une aiguille selon l'invention, en la comparant avec la configuration d'une fibre optique sans aiguille et dont l'extrémité distale présente une surface droite, c'est à dire à 90° soit perpendiculaire à l'axe de cette fibre.

Le graphique de la FIGURE 6 représente l'intensité obtenue dans de l'eau par le dispositif selon l'invention sur un domaine spectral de 430 nm à 750 nm, comparé avec un montage avec un fibre nue d'extrémité distale à 90°.

Le graphique de la FIGURE 7 représente l'intensité obtenue par le dispositif selon l'invention sur le même domaine spectral après soustraction du bruit de fond, pour un échantillon tumoral, comparé avec un montage avec un fibre seule d'extrémité distale à 90°. Cette figure montre que la forme des signaux mesurés dans un échantillon de tissu humain est inchangée quelle que soit la sonde utilisée, avec une facteur d'atténuation environ de deux.

L'expérimentation montre que le signal de la fibre polie à 15° sous aiguille est suffisamment fort pour être détecté, et peut donc être utilisé pour l'application visée.

Ces résultats ont été obtenus avec une puissance calculée pour respecter les critères de sécurité d'emploi du laser in vivo. Ces critères dépendant de la surface de contact de l'extrémité distale de la fibre avec les tissus à explorer, or le fait d'utiliser un biseau augmente cette surface. Il est ainsi possible d'augmenter de la puissance du laser, et compenser en partie la baisse du signal constatée avec la fibre biseautée sous aiguille.

Le rapport signal bruit (amplitude signal/amplitude bruit) pour des mesures individuelles est compris entre 6 et 9 pour une durée d'acquisition de une seconde, ce qui fournit des résultats suffisamment significatifs pour envisager la possibilité d'une mesure par point.

L'expérimentation semble ainsi confirmer l'efficacité de l'architecture de mesure avec une fibre aiguisée et dans sous aiguille comme décrite ici, plus particulièrement en combinaison avec le dispositif d'injection et récupération de lumière, par exemple en utilisant l'un ou l'autre des critères d'intensité ou de décalage spectral ou des deux.

Bien sûr, l'invention n'est pas limitée aux exemples qui viennent d'être décrits et de nombreux aménagements peuvent être apportés à ces exemples sans sortir du cadre de l'invention tel que défini dans les revendications.

## Revendications

1. Dispositif d'observation ou de diagnostic in vivo en profondeur d'un tissu biologique compact ou d'un organe vivant, notamment d'une tumeur, comprenant une sonde (10) munie d'une extrémité proximale du côté de l'opérateur et une extrémité distale (11) du côté du tissu à observer, et comprenant une aiguille creuse (3) se terminant à l'extrémité distale par une pointe coupante (4) apte à pénétrer la surface du tissu à observer et à s'enfoncer à l'intérieur dudit tissu, et comprenant des moyens d'injection et de récupération de lumière (6), **caractérisé en ce que**
- l'aiguille entoure de façon solidaire une fibre optique unique (2), de préférence multimode, dont l'extrémité proximale (22) est connectée ou est agencée pour être connectée auxdits moyens d'injection et de récupération de lumière (6) ;
- l'extrémité distale (21) de ladite fibre optique unique (2) présente un profil transversal dans la continuité de la pointe (4) de l'aiguille (3) en un biseau simple faisant avec l'axe (A3) de l'aiguille un angle compris entre 10° et 25° ;
- les moyens d'injection et de récupération de lumière (6) sont agencés pour injecter un signal lumineux, dit aller (601), dans l'extrémité proximale de la fibre optique (2), et recevoir de l'extrémité proximale de ladite fibre optique (2) au moins un signal lumineux dit retour (602) de fluorescence endogène générée par l'excitation due à ladite lumière injectée ; et
- lesdits moyens d'injection et de récupération (6) comprennent un dispositif d'analyse (60) agencé pour mesurer ledit signal retour (602).

2. Dispositif selon la revendication précédente, **caractérisé en ce que** les moyens d'injection de lumière injectent une lumière d'une longueur d'onde comprise entre 370 et 420 nanomètres.

3. Dispositif selon l'une quelconque des revendications précédentes **caractérisé en ce que** les moyens d'injection de lumière comprennent une source (61) de lumière laser, telle qu'une diode laser à 405 nm, pour générer la lumière injectée (601) qui est focalisée par un objectif (63) jusque dans la fibre optique (2) après être passée au travers d'un élément séparateur (62)) ; et
**en ce que** le signal de retour (602) provenant de la même fibre unique (2) est collecté par le même objectif (63) ayant servi à l'injection, puis redirigé vers le même élément séparateur (62) qui le renvoie vers le dispositif d'analyse (60).

4. Dispositif selon l'une quelconque des revendications précédentes, **caractérisé en ce que** le dispositif d'analyse (60) comprend un filtre interférentiel (64) coupant la longueur d'onde de la lumière d'excitation (601) et laissant passer les longueurs d'ondes supérieures, et un objectif (65) focalisant tout ou partie de la lumière de retour (602) sur l'entrée d'un spectrophotomètre (66).

5. Dispositif selon l'une quelconque des revendications précédentes, **caractérisé en ce que** le dispositif d'analyse (60) comprend des moyens de séparation selon la longueur d'onde (67), à longueur d'onde de séparation de 5 à 30 nm supérieure à celle de la lumière injectée (601), disposée pour séparer tout ou partie du signal retour (602) en
- une première voie (6021) dans laquelle la lumière correspond aux longueurs d'onde de la fluorescence endogène des tissus observés (91) et est focalisée par un objectif (651) sur un premier photodétecteur (661) ; et
- une deuxième voie (6022) dans laquelle la lumière correspond à la longueur de la lumière injectée (601) et est focalisée par un objectif (652) sur un deuxième photodétecteur (662).

6. Dispositif selon l'une quelconque des revendications précédentes, **caractérisé en ce que** la sonde (10) comporte une résine transparente réunissant en une même surface continue et étanche l'extrémité distale (21) de la fibre optique (2) avec la pointe (4) de l'aiguille (3).

7. Dispositif selon l'une quelconque des revendications précédentes, **caractérisé en ce que** la sonde comprend une aiguille présentant des dimensions inférieures ou égales aux aiguilles de taille 23G.

8. Dispositif selon l'une quelconque des revendications précédentes, **caractérisé en ce que** la fibre (2) à l'intérieur de l'aiguille (3) est une fibre de silice, optimisée pour la transmission dans le spectre bleu-vert et de coeur de l'ordre de 200 µm.

9. Dispositif selon l'une des revendications précédentes **caractérisé en ce qu'**il comprend des moyens de télémétrie optique (7) qui sont fixés sur la partie extérieure de la sonde (10) et sont agencés pour mesurer et transmettre la profondeur d'enfoncement (P10) de ladite sonde à l'intérieur du tissu (9), à partir d'une mesure de la distance (D7) jusqu'à la surface extérieure (90) dudit tissu.

10. Dispositif selon la revendication précédente, **caractérisé en ce que** les moyens de télémétrie optique (7) comprennent au moins une paire de fibres optiques juxtaposées, parmi lesquelles une première fibre de télémétrie envoie parallèlement à l'axe (A3) de l'aiguille (3) un faisceau de lumière vers la surface extérieure (90) du tissu (9) à observer, et une seconde fibre de télémétrie récupère la lumière rétrodiffusée par ladite surface (90) et est connectée à un phototransistor ou une photodiode qui mesure la fraction rétrodiffusée par la surface (90) au sein du flux de lumière émise par la première fibre de télémétrie, fournissant ainsi une mesure de la distance (D7) jusqu'à ladite surface extérieure (90).

11. Procédé de fabrication d'une sonde (10) d'un dispositif selon l'une quelconque des revendications précédentes, **caractérisé en ce qu'**il comprend les étapes suivantes :
- insertion et collage d'une fibre optique (2) dans le creux d'une aiguille creuse (3) se terminant par une pointe coupante (4), et
- polissage de ladite fibre optique (2) à son extrémité distale (11) de façon à épouser la forme tranchante de la pointe de l'aiguille (3).

## Patentansprüche

1. Vorrichtung zur tiefen Beobachtung bzw. in-vivo-Diagnostik eines kompakten, biologischen Gewebes oder eines lebenden Organs, insbesondere eines Tumors, umfassend eine Sonde (10) mit einem proximalen Ende auf der Seite des Bedieners und einem distalen Ende (11) auf der Seite des zu beobachtenden Gewebes und umfassend eine Hohlnadel, (3) deren distales Ende mit einer Schneidspitze (4) endet, die zum Durchdringen der Oberfläche des zu beobachtenden Gewebes und zum Eindringen ins Innere des genannten Gewebes geeignet ist, und umfassend Injektions- und Sammlungsmittel von Licht (6), **dadurch gekennzeichnet, dass**
- die Nadel eine einzelne, bevorzugt multimode Glasfaser (2) solidarisch umhüllt, deren proximales Ende (22) angeschlossen bzw. derart angeordnet ist, um mit den genannten Injektions- und Sammlungsmitteln von Licht (6) angeschlossen zu werden;
- das distale Ende (21) der einzelnen Glasfaser (2) ein einfach abgeschrägtes Querprofil in der Verlängerung der Spitze (4) der Nadel (3) aufweist, das mit der Achse (A3) der Nadel einen Winkel zwischen 10° und 25° bildet;
- die Injektions- und Sammlungsmittel von Licht (6) derart ausgebildet sind, um einen sogenannten abgehenden Lichtsignal (601) in das proximale Ende der Glasfaser (2) zu injizieren und vom proximalen Ende der Glasfaser (2) zumindest einen sogenannten zurückgesandten Lichtsignal (602) von endogener Fluoreszenz zu empfangen, die durch die Erregung mit dem injizierten Licht erzeugt wird; und
- die Injektions- und Sammlungsmittel (6) eine Analysevorrichtung (60) umfassen, die zur Messung des genannten Rücksignals (602) ausgebildet ist.

2. Vorrichtung nach dem vorhergehenden Anspruch, **dadurch gekennzeichnet, dass** die Lichtinjektionsmittel ein Licht mit einer Wellenlänge zwischen 370 und 420 Nanometer injizieren.

3. Vorrichtung nach einem der vorhergehenden Ansprüche, **dadurch gekennzeichnet, dass** die Lichtinjektionsmittel eine Laserlichtquelle (61) wie zum Beispiel eine Laserdiode von 405 nm zur Erzeugung des injizierten Lichts (601) umfassen, das durch ein Objektiv (63) bis in die Glasfaser (2) fokussiert wird, nachdem es durch ein Trennelement (62) geleitet wurde; und dadurch, dass der Rücksignal (602) aus derselben einzelnen Glasfaser (2) durch dasselbe Objektiv (63) gesammelt wird, das zur Injektion gedient hat, dann zu demselben Trennelement (62) zurückgeleitet wird, das ihn zur Analysevorrichtung (60) zurücksendet.

4. Vorrichtung nach einem der vorhergehenden Ansprüche, **dadurch gekennzeichnet, dass** die Analysevorrichtung (60) ein Interferenzfilter (64), das die Wellenlänge des Erregungslichts (601) abschneidet und die höheren Wellenlängen durchlässt, sowie ein Objektiv (65), das das rückgesandte Licht (602) ganz oder teilweise auf den Eingang eines Spektralphotometers (66) fokussiert, umfasst.

5. Vorrichtung nach einem der vorhergehenden Ansprüche, **dadurch gekennzeichnet, dass** die Analysevorrichtung (60) Trennmittel entsprechend der Wellenlänge (67) mit Trennwellenlänge von 5-30 nm über die des injizierten Lichts (601) umfasst, welche Trennwellenlänge zur Trennung des rückgesandten Signals (602) angeordnet ist, und zwar in
- einen ersten Kanal (6021), in welchem das Licht den Wellenlängen der endogenen Fluoreszenz der beobachten Gewebe (91) entspricht und durch ein Objektiv (651) auf einen ersten Photodetektor (661) fokussiert wird; und
- einen zweiten Kanal (6022), in welchem das Licht der Länge des injizierten Lichts (601) entspricht und durch ein Objektiv (652) auf einen zweiten Photodetektor (662) fokussiert wird.

6. Vorrichtung nach einem der vorhergehenden Ansprüche, **dadurch gekennzeichnet, dass** die Sonde (10) ein durchsichtiges Harz umfasst, das in einer selben, durchgehenden und undurchlässigen Oberfläche das distale Ende (21) der Glasfaser (2) mit der Spitze (4) der Nadel (3) vereint.

7. Vorrichtung nach einem der vorhergehenden Ansprüche, **dadurch gekennzeichnet, dass** die Sonde eine Nadel umfasst, die Abmessungen kleiner oder gleich als die Nadeln der Größe 23G aufweist.

8. Vorrichtung nach einem der vorhergehenden Ansprüche, **dadurch gekennzeichnet, dass** die Faser (2) im Inneren der Nadel (3) eine Quarzfaser ist, die für die Übertragung im blau-grünen Spektrum optimiert und mit Kern von ca. 200µm ist.

9. Vorrichtung nach einem der vorhergehenden Ansprüche, **dadurch gekennzeichnet, dass** sie optische Telemetriemittel (7) umfasst, die auf dem Außenteil der Sonde (10) befestigt und dazu ausgebildet sind, die Einsenkungstiefe (P10) der Sonde im Inneren des Gewebes (9) anhand einer Messung der Entfernung (D7) zur Außenfläche (90) des Gewebes zu messen und zu übertragen.

10. Vorrichtung nach dem vorherigen Anspruch, **dadurch gekennzeichnet, dass** die optischen Telemetriemittel (7) mindestens ein Paar nebeneinanderlaufender Glasfaser umfasst, unter denen eine erste Telemetriefaser parallel zur Achse (A3) der Nadel (3) einen Lichtstrahl zur Außenfläche (90) des zu beobachtenden Gewebes (9) sendet und eine zweite Telemetriefaser das durch die genannte Oberfläche (90) rückgestreute Licht auffängt und mit einem Phototransistor oder einer Photodiode verbunden ist, die den durch die Oberfläche (90) rückgestreuten Anteil innerhalb des durch die erste Telemetriefaser ausgestrahlten Lichtstroms misst und somit einen Messwert für die Entfernung (D7) zur genannten Außenfläche (90) ausgibt.

11. Verfahren zur Herstellung einer Sonde (10) einer Vorrichtung nach einem der vorhergehenden Ansprüche, **dadurch gekennzeichnet, dass** es folgende Arbeitsschritte umfasst:
- Einsetzen und Kleben einer Glasfaser (2) in den Hohlraum einer Hohlnadel (3), die mit einer Schneidspitze (4) endet, und
- Schleifen der Glasfaser (2) an ihrem distalen Ende (11), sodass sie die schneidende Form der Spitze der Nadel (3) annimmt.

## Claims

1. A device for the in-depth in vivo observation or diagnostics of a compact biological tissue or a living organ, in particular a tumour, comprising a probe (10) provided with a proximal end on the operator side and a distal end (11) on the side of the tissue to be observed, and comprising a hollow needle (3) ending at the distal end in a cutting point (4) able to penetrate the surface of the tissue to be observed and to sink within said tissue, and comprising injection and recovery light means (6), **characterised in that**
- the needle integrally surrounds a single optical fibre (2), preferably of the multimode type, the proximal end (22) of which is connected or is arranged to be connected to said light injection and recovery means (6) ;
- the distal end (21) of the single optical fibre (2) has a transverse profile in the continuity of the point (4) of the needle (3) with a single bevel forming with the axis (A3) of the needle an angle between 10° and 25°;
- the injection and recovery light means (6) are arranged to inject a so-called feed forward light signal (601), into the proximal end of the optical fibre (2), and to receive out of the proximal end of said optical fibre (2) at least one so-called feedback light signal (602) of endogenous fluorescence generated by the excitation due to said injected light; and
- said injection and recovery light means (6) comprise an analysing device (60) arranged for measuring said feedback signal (602).

2. The device according to the preceding claim, **characterised in that** the light injection means inject a light with a wavelength between 370 and 420 nanometres.

3. The device according to any of the preceding claims, **characterised in that** the light injection means comprise a laser light source (61), such as a laser diode at 405 nm, for generating the injected light (601) which is focused by an objective lens (63) to inside the optical fibre (2) after passing through a separating element (62); and
**in that** the feedback signal (602) coming from the same single fibre (2) is collected by the same objective lens (63) which was used for injection, and then redirected to the same separating element (62) which sends it back to the analysing device (60).

4. The device according to any of the preceding claims, **characterised in that** the analysing device (60) comprises an interference filter (64) cutting off the wavelength of the excitation light (601) and passing the higher wavelengths, and an objective lens (65) focusing part or all of the feedback light (602) onto the inlet of a spectrophotometer (66).

5. The device according to any of the preceding claims, **characterised in that** the analysing device (60) comprises separating means according to the wavelength (67), with a separating wavelength 5 to 30 nm higher than that of the injected light (601), provided to separate part or all of the feedback signal (602) into
- a first path (6021) wherein the light corresponds to the wavelengths of the endogenous fluorescence of the observed tissues (91) and is focused by an objective lens (651) onto a first photodetector (661); and
- a second path (6022) wherein the light corresponds to the length of the injected light (601) and is focused by an objective lens (652) onto a second photodetector (662).

6. The device according to any of the preceding claims, **characterised in that** the probe (10) includes a transparent resin gathering in a same continuous tight surface the distal end (21) of the optical fibre (2) with the point (4) of the needle (3).

7. The device according to any of the preceding claims, **characterised in that** the probe comprises a needle having dimensions equal to or lower than 23 gauge needles.

8. The device according to any of the preceding claims, **characterised in that** the fibre (2) within the needle (3) is a silica fibre, optimized for the transmission in the blue green spectrum and with a core in the order of 200 µm.

9. The device according to one of any of the preceding claims, **characterised in that** it comprises optical telemetry means (7) which are attached to the outer part of the probe (10) and arranged to measure and transmit the sinking depth (P10) of said probe into the tissue (9), from a measure of the distance (D7) up to the outer surface (90) of said tissue.

10. The device according to the preceding claim, **characterised in that** the optical telemetry means (7) comprise at least one pair of juxtaposed optical fibres, among which a first telemetry fibre sends in parallel to the axis (A3) of the needle (3) a light beam to the outer surface (90) of the tissue (9) to be observed, and a second telemetry fibre recovers the light backscattered by said surface (90) and is connected to a phototransistor or a photodiode which measures the fraction backscattered by the surface (90) within the luminous flux emitted by the first telemetry fibre, thereby providing a measure of the distance (D7) up to said outer surface (90).

11. Method for producing a probe (10) according to any of the preceding claims, **characterised in that** it comprises the following steps:
- inserting and glueing a single optical fibre (2) within the cavity of a hollow needle (3) ending in a cutting point (4), and
- polishing said optical fibre (2) at its distal end (11) so that it matches to the cutting shape of the point of the needle (3).
